# EUROPEAN PATENT APPLICATION

(11) **EP 4 180 818 A1**
(43) Date of publication of application: **17.05.2023**
(21) Application number: 21835929.7
(22) Date of filing: 25.02.2021
(51) Int. Cl.: G01N 35/00

(54) **AUTOMATED ANALYZER AND AUTOMATED ANALYZER MAINTENANCE METHOD**

(30) Priority: 07.07.2020 JP 2020117147
(71) Applicant: HITACHI HIGH-TECH CORPORATION, Tokyo 105-6409 (JP)
(72) Inventor: SHIROTANI, Masahiro, Tokyo 105-6409 (JP); YASUI, Akihiro, Tokyo 105-6409 (JP); SETOMARU, Takeshi, Tokyo 105-6409 (JP)
(74) Representative: Strehl Schübel-Hopf & Partner
(86) International application number: PCT/JP2021/007120
(87) International publication number: WO 2022/009463

(57) **Abstract**

Provided are an automatic analyzer and a maintenance method for an automatic analyzer that can reduce an operation of a user relating to maintenance in a plurality of maintenance operation processes. The automatic analyzer includes an analysis unit 111 including a plurality of components and configured to analyze a specimen, and an overall control unit 134 configured to control an operation of the analysis unit 111. The analysis unit 111 is provided with a sensor that monitors states of the components, and when the sensor detects that an operator executed a specific operation on the components, the overall control unit 134 requests confirmation as to whether execution status of a maintenance operation needs to be updated.

## Description

### Technical Field

The present invention relates to an automatic analyzer that performs component analysis of a biological sample (hereinafter, simply referred to as a specimen) such as serum, plasma, or urine, and a maintenance method for an automatic analyzer.

### Background Art

As an example of an automatic analyzer capable of reducing a maintenance operation without hindering measurement, PTL 1 discloses that an automatic analyzer includes: an analysis section that includes analysis units that dispense a test sample and a reagent and measure a mixed solution thereof; an analysis control unit that controls operations of the analysis units; detectors that detect liquid levels when the analysis units come into contact with any one liquid of the test sample, the reagent, or the mixed solution thereof; and a history data creation unit that creates maintenance history data, which is a history of maintenance, based on a detection signal detected by detection corresponding to an analysis unit of a maintenance management target, in which when measurement is not performed by the analysis section in response to a predetermined operation from an operation unit, a maintenance promotion message is displayed on a display unit while the analysis unit of the maintenance management target is moved to a preset cleaning position.

### Citation List

### Patent Literature

PTL 1: JP2009-168491A

### Summary of Invention

### Technical Problem

In the automatic analyzer, a reagent that specifically reacts with a specific component contained in the specimen is added to and reacts with the specimen, and transmitted light, scattered light, chemiluminescence, and electrochemical luminescence are measured to perform a qualitative or quantitative analysis for the specific component contained in the specimen.

In the automatic analyzer, in order to make a state of the analyzer suitable for analysis, the maintenance operation is periodically performed to appropriately manage the state of the analyzer. Accordingly, the reliability of an analysis result is improved and analysis accuracy is stabilized.

PTL 1 discloses a technique for promoting maintenance without hindering measurement by moving the analysis units that dispense the specimen and the reagent and measure the mixed solution thereof to the preset cleaning position when the measurement is not performed in the maintenance operation performed manually.

PTL 1 discloses a technique for only prompting a user to perform maintenance, not prompting the user to perform the maintenance operation at a convenient timing. PTL 1 discloses that a message prompting the maintenance operation is displayed at a predetermined time interval, when the power of the analyzer is turned on, when the power is turned off, when the analyzer is cleaned, or the like, but the user does not necessarily perform the maintenance operation at such timing. Therefore, it may be necessary to interrupt a predetermined operation or change an operation order for a maintenance operation, and a more user-friendly technique is expected.

In PTL 1, it is not possible to determine whether the maintenance operation is actually performed. Accordingly, it is clear that there is room for improvement in prompting an excessive maintenance operation by recommending maintenance in which the history is updated assuming that the maintenance operation is performed even though the operation is not actually performed, or maintenance in which the history is not updated even though the maintenance operation is performed and which is not originally necessary, and that there is room for more accurately reflecting the maintenance history.

The invention is made in view of the above problems, and provides an automatic analyzer and a maintenance method for an automatic analyzer that can reduce an operation of a user relating to maintenance in a plurality of maintenance operation processes.

### Solution to Problem

The invention includes a plurality of methods for solving the above-described problems, and as an example thereof, there is provided an automatic analyzer for analyzing a specimen. The automatic analyzer includes: an analysis unit including a plurality of components and configured to analyze the specimen; and a control unit configured to control an operation of the analysis unit. The analysis unit is provided with a sensor that monitors states of the components, and when the sensor detects that an operator performed a specific operation on the components, the control unit requests confirmation as to whether execution status of a maintenance operation needs to be updated.

### Advantageous Effects of Invention

According to the invention, in a plurality of maintenance operation processes, it is possible to reduce an operation of a user relating to maintenance. Problems, configurations, and effects other than those described above will be clarified by the following description of an embodiment.

### Brief Description of Drawings

[FIG. 1] FIG. 1 is a schematic diagram showing an example of an overall configuration of an automatic analyzer according to an embodiment of the invention.
[FIG. 2] FIG. 2 is a schematic diagram showing a dispensing unit of the automatic analyzer according to the embodiment.
[FIG. 3] FIG. 3 is a schematic diagram showing an electrolyte measurement unit of the automatic analyzer according to the embodiment.
[FIG. 4] FIG. 4 is a schematic diagram showing a configuration example of a function of an operation unit of the automatic analyzer according to the embodiment.
[FIG. 5] FIG. 5 is a diagram showing an example of a function of a device state monitoring history storage unit in the automatic analyzer according to the embodiment.
[FIG. 6] FIG. 6 is a diagram showing an example of a function of a maintenance operation history storage unit in the automatic analyzer according to the embodiment.
[FIG. 7] FIG. 7 is a diagram showing an example of a function of a user information storage unit in the automatic analyzer according to the embodiment.
[FIG. 8] FIG. 8 is a flowchart showing an example of processing contents in a condition determination unit in FIG. 4.
[FIG. 9] FIG. 9 is a diagram showing an example of a confirmation screen (GUI) for prompting confirmation of a maintenance execution status in the automatic analyzer according to the embodiment.
[FIG. 10] FIG. 10 is a diagram showing another example of the confirmation screen (GUI) for prompting confirmation of the maintenance execution status in the automatic analyzer according to the embodiment.
[FIG. 11] FIG. 11 is a diagram showing an example of a selection screen (GUI) for selecting automatic execution and simultaneous execution of operations after maintenance in the automatic analyzer according to the embodiment.
[FIG. 12] FIG. 12 is a diagram showing an example of an absorbance measurement result in the automatic analyzer of the embodiment.

### Description of Embodiments

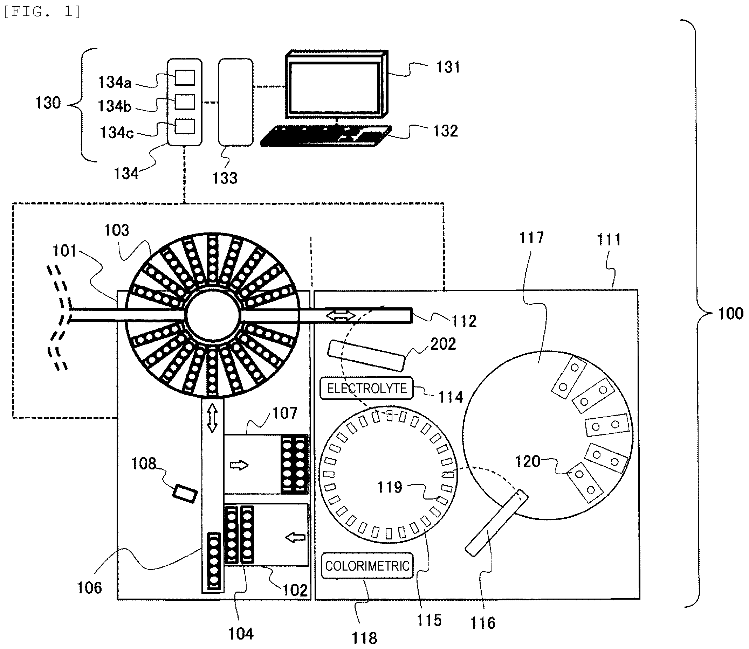

An embodiment of an automatic analyzer and a maintenance method for an automatic analyzer of the invention will be described with reference to FIGS. 1 to 12.

In the following embodiment, description may be divided into a plurality of sections or embodiments for convenience if necessary. Unless particularly specified, the sections or embodiments are not independent of each other, but have a relation in which one section or embodiment is a modification, detailed description, supplementary description, or the like of a part or all of another section or embodiment.

In addition, in the following embodiment, when a number and the like (including the number, a numerical value, an amount, a range, and the like) of an element are referred to, the number and the like are not limited to specific numbers, and may be equal to or greater than or equal to or less than the specific numbers, unless otherwise specified or clearly limited to the specific numbers in principle.

Further, in the following embodiment, it is needless to say that constituent elements (including element steps and the like) are not always indispensable unless otherwise stated or except in the case where the components are apparently indispensable in principle. Similarly, in the following embodiment, shapes, positional relation, or the like of the constituent elements or the like include those substantially approximate or similar to the shapes or the like unless otherwise particularly specified or when it is clearly considered that this is not the case in principle.

The same applies to the numerical value and the range.

### <Overall Configuration and Operation of Automatic Analyzer>

First, an overall configuration and an operation of an automatic analyzer according to the present embodiment will be described with reference to FIG. 1. FIG. 1 is a diagram schematically showing the overall configuration of the automatic analyzer according to the present embodiment.

An automatic analyzer 100 shown in FIG. 1 is a device for analyzing a specimen, and includes a conveyance unit 101, an analysis unit 111, and an operation unit 130.

The conveyance unit 101 is a unit that loads a specimen rack 104 on which one or more specimen containers containing specimens to be analyzed such as blood and urine are mounted into the automatic analyzer 100, collects and conveys the specimen rack 104 in the automatic analyzer 100, and supplies the specimens to the analysis unit 111.

The conveyance unit 101 includes a rack buffer 103, a rack supply tray 102, a rack storage tray 107, and a conveyance line 106.

In the conveyance unit 101, the specimen rack 104 disposed on the rack supply tray 102 is conveyed to the rack buffer 103 by the conveyance line 106. A specimen information reading device 108 such as a sensor is provided in the middle of the conveyance line 106, and reads and recognizes an identification medium such as an RFID or a barcode provided on the specimen rack 104 to be described later. By providing the identification medium not only in the specimen rack 104 but also in the specimen containers, it is possible to recognize the identification information for each specimen container.

The rack buffer 103 has a rotor structure that performs circular motion, and an outer circumference thereof has slots that radially hold a plurality of specimen racks 104, on each of which a plurality of specimen containers are mounted in concentric circles. When the slots are rotated by a motor, any specimen rack 104 is carried in or carried out to or from the analysis unit 111 of a request destination. With such a structure, it is possible to process the specimen racks 104 without being limited to an order of entering. That is, when a specimen rack has a high priority, the specimen rack can be processed first.

The conveyance line 106 is connected to a certain point on a radial circumference of the rack buffer 103 to carry the specimen rack 104 in and out. Assuming that this point is at a position of 0° on the circumference, a specimen dispensing line 112 for drawing the specimen rack 104 into the analysis unit 111 to be described later is connected to a position of 90° on the circumference from a position to which the conveyance line 106 is connected to carry the specimen rack 104 in and out.

After finishing dispensing in the analysis unit 111, the specimen rack 104 can wait for an output of a measurement result in the rack buffer 103 and perform processing such as automatic retest as necessary. Further, when the processing is finished, the specimen rack 104 is conveyed to the rack storage tray 107 via the conveyance line 106.

The analysis unit 111 is a unit that performs a measurement operation of a measurement item requested for a specimen and outputs a measurement result, and is connected to the conveyance unit 101.

The analysis unit 111 includes a plurality of components such as a reaction disk 115, a reagent disk 117, the specimen dispensing line 112, a reagent dispensing nozzle 116, a specimen dispensing nozzle 202, a colorimetric measurement unit 118, and an electrolyte measurement unit 114.

Reaction cells 119 are arranged on a circumference of the reaction disk 115. The specimen dispensing line 112, into which the specimen rack 104 with the specimen containers placed thereon is carried, is installed near the reaction disk 115. Further, a cell cover (not shown) is installed on an upper portion of the reaction disk 115, and a sensor for detecting opening and closing of the cell cover is installed.

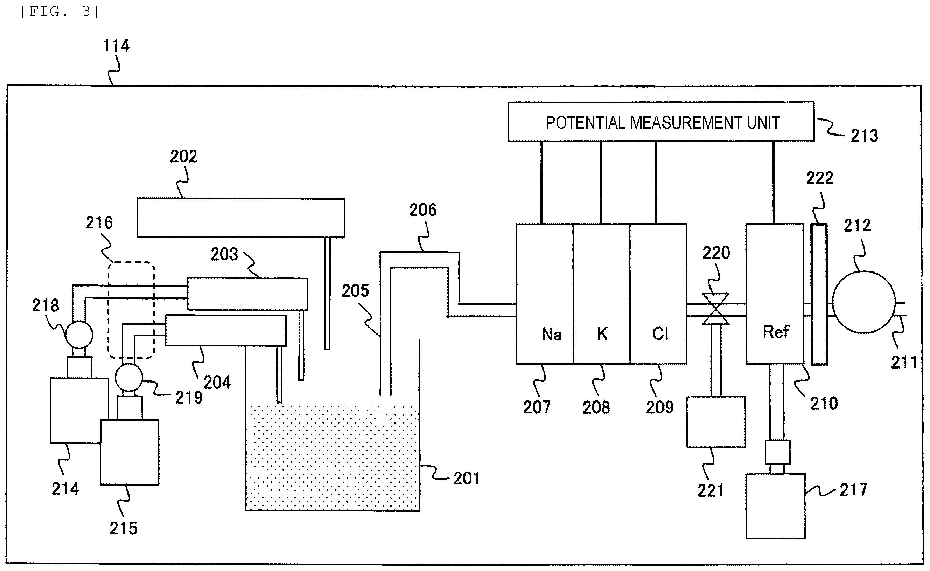

The specimen dispensing nozzle 202, which can be rotated and moved up and down, is installed between the reaction disk 115 and the specimen dispensing line 112. The specimen dispensing nozzle 202 moves while drawing an arc around a rotation axis to dispense the specimen from the specimen rack 104 to the reaction containers on the reaction disk 115 or a dilution tank 201 (see FIG. 3) in the electrolyte measurement unit 114.

The reagent disk 117 is a storage cabinet on whose circumference a plurality of reagent bottles 120 containing reagents therein can be mounted. The reagent disk 117 is kept cold.

The reagent dispensing nozzle 116, which can be rotated and moved up and down, is installed between the reaction disk 115 and the reagent disk 117. The reagent dispensing nozzle 116 moves while drawing an arc around a rotation axis to access an inside of the reagent disk 117 from a reagent dispensing nozzle aspiration port and dispense a reagent from a reagent bottle to a reaction container.

Further, cleaning tanks (not shown) are installed in operation ranges of the reagent dispensing nozzle 116 and the specimen dispensing nozzle 202, respectively.

The electrolyte measurement unit 114 and the colorimetric measurement unit 118 are disposed around the reaction disk 115.

The electrolyte measurement unit 114 is an analysis unit that measures an electrolyte concentration in a specimen by using ion selective electrodes. Details thereof will be described later with reference to FIG. 3.

The colorimetric measurement unit 118 is an analysis unit that performs analysis of biochemical components in a specimen by measuring absorbance of a reaction solution generated by performing mixing and reaction in a reaction container on the reaction disk 115, and executes analysis of an analysis item having a measurement principle different from that of the electrolyte measurement unit 114. The colorimetric measurement unit 118 includes a light source, a spectrophotometer, and the like.

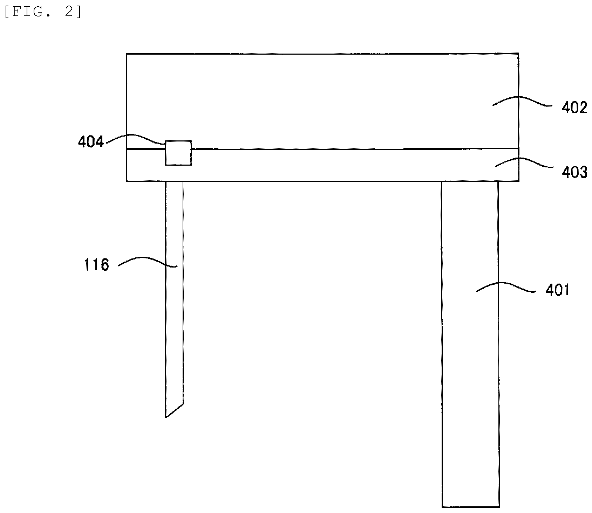

The analysis unit 111 described above is provided with a plurality of sensors that monitor the states of the components. For example, in the case of the reagent dispensing nozzle 116 as shown in FIG. 2, an opening and closing sensor 404 is provided to detect whether an arm cover upper portion 402 is detached from an arm cover lower portion 403 provided on an upper side of a vertical rotation mechanism 401. A monitoring step of monitoring the states of the components constituting the analysis unit 111 is executed by various sensors that include the opening and closing sensor 404 and are provided in the analysis unit 111 that analyzes the specimen.

Other examples of the sensors include, in addition to the opening and closing sensor 404, a sensor that detects whether various covers provided in each mechanism in the automatic analyzer 100 are opened or closed and attached or detached, a sensor that detects whether an operator accesses the analysis unit 111 from the end of an analysis operation to the start of an analysis preparation operation, and a sensor that detects whether a component is attached or detached.

The operation unit 130 (a computer system) is a unit that collectively controls information of the entire automatic analyzer 100, and includes a display unit 131, an input unit 132, a recording unit 133, and an overall control unit 134. The operation unit 130 is connected to the analysis unit 111 and the conveyance unit 101 by a wired or wireless network line.

The display unit 131 is a unit that displays various screens such as an operation screen for ordering measurement items to be measured for a specimen to be measured and a screen for confirming a measurement result, and includes a liquid crystal display or the like. The display unit 131 is not necessarily a liquid crystal display, and may be replaced with a printer or the like. The display and the printer or the like can be used as a touch panel type display that also serves as the input unit 132 described later.

The input unit 132 is a unit that inputs various parameters, settings, measurement results, measurement request information, analysis start and stop instructions, and the like based on an operation screen displayed on the display unit 131, and includes a keyboard, a mouse, or the like.

The recording unit 133 is a unit that stores a time chart and operation parameters necessary for an operation of each device that constitutes the automatic analyzer 100, various information for identifying a specimen, measurement results, and the like, and includes a storage medium such as a semiconductor memory such as a flash memory or a magnetic disk such as an HDD.

The overall control unit 134 controls an operation of the entire automatic analyzer 100 including the analysis unit 111, and includes a conveyance unit controller 134a, an analysis unit controller 134b, and an operation recording unit 134c.

The conveyance unit controller 134a controls an operation of conveying an appropriate specimen rack 104 from the rack buffer 103 to the specimen dispensing line 112 and a conveyance operation of returning the specimen rack 104 from the specimen dispensing line 112 to the rack buffer 103.

The analysis unit controller 134b is connected to devices in the analysis unit 111, and controls an analysis operation by constituent devices of the electrolyte measurement unit 114 and the colorimetric measurement unit 118.

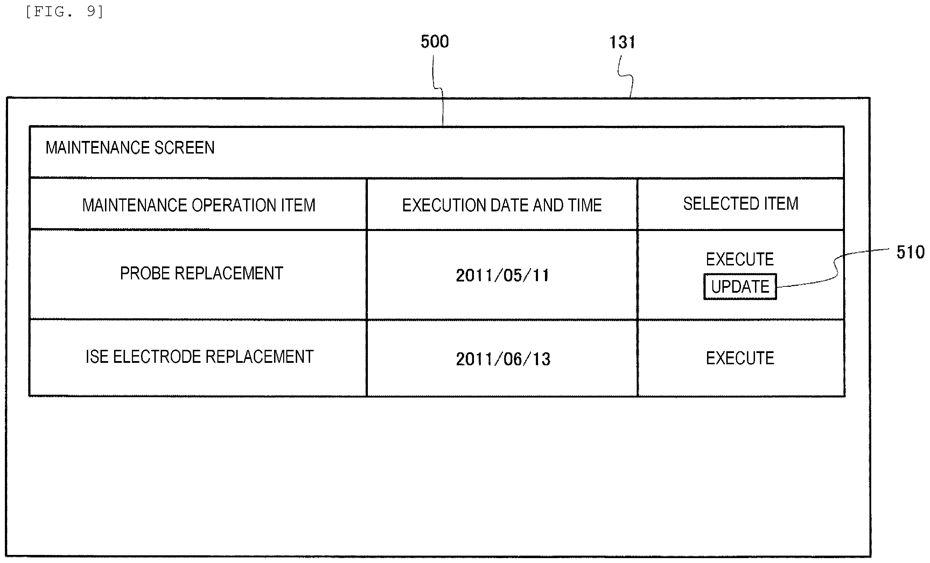

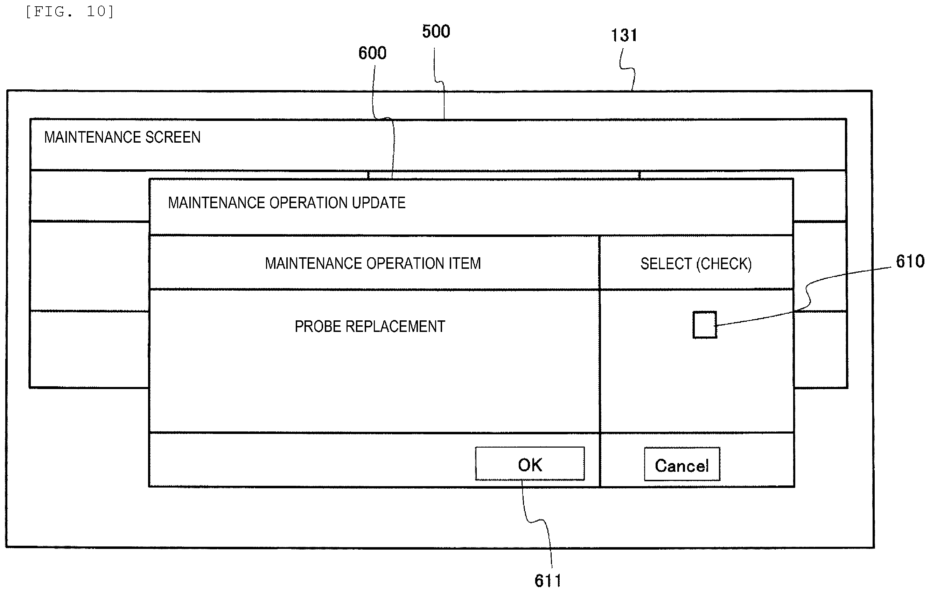

In the present embodiment, when the sensors detect that the operator executed a specific operation on the components, the analysis unit controller 134b of the overall control unit 134 requests confirmation as to whether execution status of a maintenance operation needs to be updated (confirmation step). The confirmation as to whether execution status of a maintenance operation needs to be updated is performed with an operation screen displayed on the display unit 131 as shown in FIGS. 9 and 10. Details thereof will be described later.

The operation recording unit 134c calculates a concentration of a specific component in a measurement target based on absorbance or the like measured by the colorimetric measurement unit 118, and calculates an ion concentration of the measurement target based on a potential or the like measured by the electrolyte measurement unit 114. A photometer (not shown) is installed in the colorimetric measurement unit.

The conveyance unit controller 134a, the analysis unit controller 134b, and the operation recording unit 134c in the overall control unit 134 may be implemented by using a general-purpose computer, or may be implemented as a function of a program executed on a computer.

That is, processing of the conveyance unit controller 134a, the analysis unit controller 134b, and the operation recording unit 134c may be implemented by storing program codes in a recording unit such as a memory and executing program codes by a processor such as a central processing unit (CPU) .

The conveyance unit controller 134a, the analysis unit controller 134b, and the operation recording unit 134c may be implemented by hardware such as a dedicated circuit board.

In the present embodiment, the analysis unit in which the electrolyte measurement unit 114 and the colorimetric measurement unit 118 are provided simultaneously is described, and the analysis unit may be the electrolyte measurement unit 114 or the colorimetric measurement unit 118 alone. The analysis unit is not limited to the electrolyte measurement unit 114 that measures an electrolyte item or the colorimetric measurement unit 118 that measures a biochemical item, and can use a measurement unit or the like that measures an immunity item.

Further, a case where the automatic analyzer 100 includes one analysis unit 111 is described, and two or more analysis units can be provided. In this case, a type of the analysis unit is not particularly limited as well, and one or more types of analysis units such as a biochemical analysis unit, an immunity analysis unit, and a blood coagulation analysis unit can be provided.

A case where the automatic analyzer 100 includes the conveyance unit 101 is described, but the conveyance unit is not necessary, and the automatic analyzer can include an analysis unit and an operation unit.

Next, an outline of a mechanism operation of the automatic analyzer 100 shown in FIG. 1 will be described.

The conveyance unit 101 sends out the specimen racks 104 disposed on the rack supply tray 102 of the automatic analyzer 100 onto the conveyance line 106 one rack at a time, and carries the specimen racks 104 into the rack buffer 103. The specimen rack 104 conveyed to the rack buffer 103 is conveyed to the specimen dispensing line 112 of the analysis unit 111.

When the specimen rack 104 reaches the specimen dispensing line 112, the specimen dispensing nozzle 202 performs a dispensing operation on the specimens mounted on the specimen rack 104 in accordance with a measurement item requested by the operation unit 130.

When the measurement item is the biochemical item, the specimen dispensing nozzle 202 discharges an aspirated specimen to a reaction container on the reaction disk 115, further adds a reagent aspirated from the reagent disk 117 by the reagent dispensing nozzle 116 to the reaction container, and stirs the reagent. Thereafter, the absorbance is measured by the colorimetric measurement unit 118, and a measurement result is transmitted to the operation recording unit 134c of the operation unit 130.

When the requested measurement item is the electrolyte item, the specimen dispensing nozzle 202 discharges the aspirated specimen to the dilution tank 201 of the electrolyte measurement unit 114, an electromotive force is measured by the ion selective electrodes 207, 208, and 209, and a measurement result is transmitted to the operation recording unit 134c of the operation unit 130. However, in a case of the electrolyte item measurement, as described above, a pre-measurement operation of measuring an electromotive force of an internal standard solution having a known concentration is necessary before dispensing a specimen.

The operation recording unit 134c of the operation unit 130 calculates a concentration of a specific component in the specimen by arithmetic processing based on the transmitted measurement result. A user is notified of an analysis result via the display unit 131, and the analysis result is recorded in the recording unit 133.

Next, an outline of the electrolyte measurement unit using the ion selective electrodes will be described with reference to FIG. 3. FIG. 3 is a schematic diagram showing an example of the electrolyte measurement unit (ISE) using the ion selective electrodes.

The electrolyte measurement unit 114 is disposed in the analysis unit 111 which automatically analyzes a specimen. A cover (not shown) is installed in an upper portion of the electrolyte measurement unit 114.

The electrolyte measurement unit 114 includes the dilution tank 201, a diluted solution dispensing nozzle 203, an internal standard solution dispensing nozzle 204, a specimen solution aspiration nozzle 205, a pipe 206, a sodium ion selective electrode 207, a potassium ion selective electrode 208, a chlorine ion selective electrode 209, a reference electrode 210, a pipe 211, a sipper pump 212, a potential measurement unit 213, a temperature control unit 216, and the like. Further, levers (not shown) for fixing the sodium ion selective electrode 207, the potassium ion selective electrode 208, the chlorine ion selective electrode 209, and the reference electrode 210 are installed.

The specimen dispensing nozzle 202 dispenses and discharges a specimen such as blood or urine to the dilution tank 201. The diluted solution dispensing nozzle 203 dispenses and discharges a diluted solution to the dilution tank 201. The internal standard solution dispensing nozzle 204 dispenses and discharges an internal standard solution to the dilution tank 201.

The diluted solution is fed from a diluted solution container 214 to the diluted solution dispensing nozzle 203 by using a diluted solution pump (DIL pump) 218. The internal standard solution is fed from an internal standard solution container 215 to the internal standard solution dispensing nozzle 204 by using an internal standard solution pump (IS pump) 219.

The temperature control unit 216 is disposed in flow paths of the diluted solution and the internal standard solution, and controls a temperature to a constant temperature (for example, 37°C) in the middle of feeding each solution. The flow path in the vicinity of the temperature control unit 216 has a flow path volume larger than those of other units, so that temperature control efficiency can also be increased.

The specimen solution aspiration nozzle 205 can be moved up and down, and aspirates a solution in the dilution tank 201 by a driving force of the sipper pump 212. The aspirated solution is introduced into flow paths of the ion selective electrodes 207, 208, and 209 through the pipe 206, and is further drained through the pipe 211.

In the electrolyte measurement unit 114, the specimen solution aspiration nozzle 205, the pipe 206, the pipe 211, and the sipper pump 212 are used as a specimen introduction unit that introduces a specimen solution containing an electrolyte. The specimen solution is introduced into the flow paths of the ion selective electrodes 207, 208, and 209 by using the specimen introduction unit.

Further, a comparison electrode solution is introduced from a comparison electrode solution container 217 to the reference electrode 210 by the pipe 211 and the sipper pump 212. The comparison electrode solution is switched, using a valve or the like, to a flow path separated from the flow paths of the internal standard solution in the ion selective electrodes 207, 208, 209, or the like so as not to contaminate the internal standard solution.

Terminals of the ion selective electrodes 207, 208, and 209 and the reference electrode 210 are connected to the potential measurement unit 213, and a potential difference among the electrodes is measured in a state where the specimen solution is introduced.

The valve 220 is provided in a flow path between the chlorine ion selective electrode 209 and the reference electrode 210, and the cleaning solution flows into a waste solution portion 221 by switching the valve 220.

### <Details of Operation Unit 130>

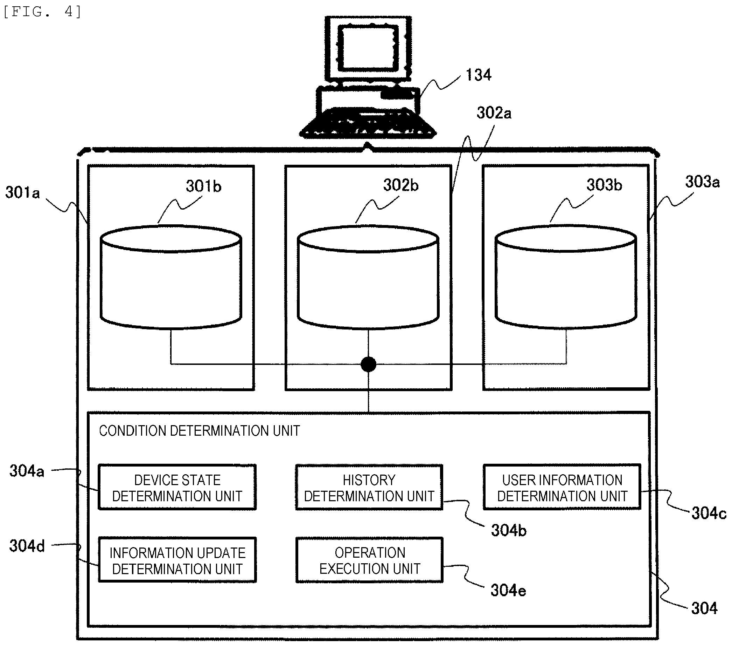

FIG. 4 is a schematic diagram showing a configuration example of the operation unit in FIG. 1. As shown in FIG. 4, the operation unit 130 includes a device state history management unit 301a, a maintenance operation history management unit 302a, a user information management unit 303a, and a condition determination unit 304.

Each of storage units (301b, 302b, 303b) is implemented mainly by a storage device such as a hard disk. The device state history management unit 301a, the maintenance operation history management unit 302a, and the user information management unit 303a are implemented by appropriately managing data in corresponding storage units by program processing using a processor, a memory, or the like, such as a database system.

The device state history management unit 301a is connected to various sensors including the opening and closing sensor 404, and when a predetermined operation is performed on each unit in the analysis unit 111 while the analysis unit 111 in FIG. 1 is stopped, the device state history management unit 301a acquires date and time of occurrence of the operation and stores the acquired date and time in the device state monitoring history storage unit 301b.

The maintenance operation history management unit 302a includes the maintenance operation history storage unit 302b, sets a condition for determining the timing of the next maintenance operation for each maintenance operation, and stores the condition in the maintenance operation history storage unit 302b.

The user information management unit 303a includes the user information storage unit 303b, sets a condition for determining whether there is a possibility of performing a maintenance operation for each user information, and stores the condition in the user information storage unit 303b.

### <Details of Device State History Management Unit 301a and Device State Monitoring History Storage Unit 301b>

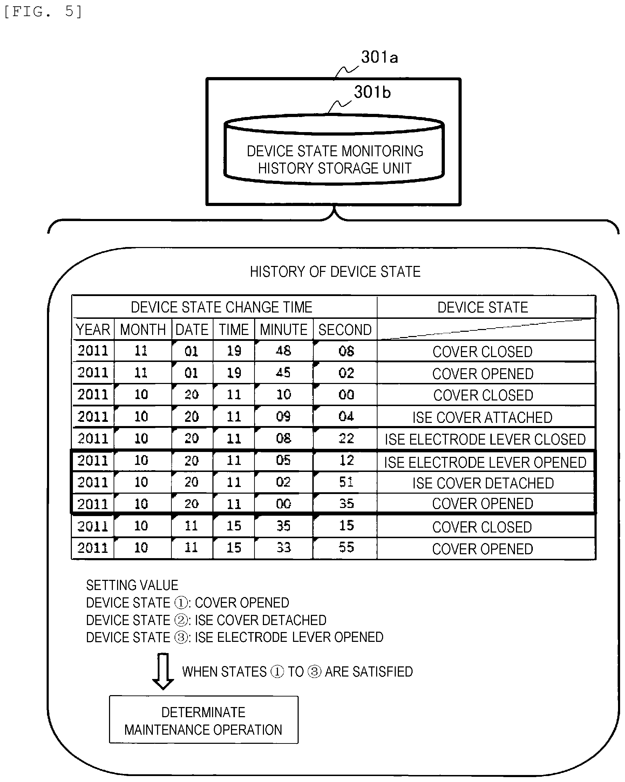

FIG. 5 is a diagram showing an example of a function of the device state monitoring history storage unit in FIG. 4. Information stored in the device state monitoring history storage unit 301b as shown in FIG. 5 is used to determine the maintenance operation.

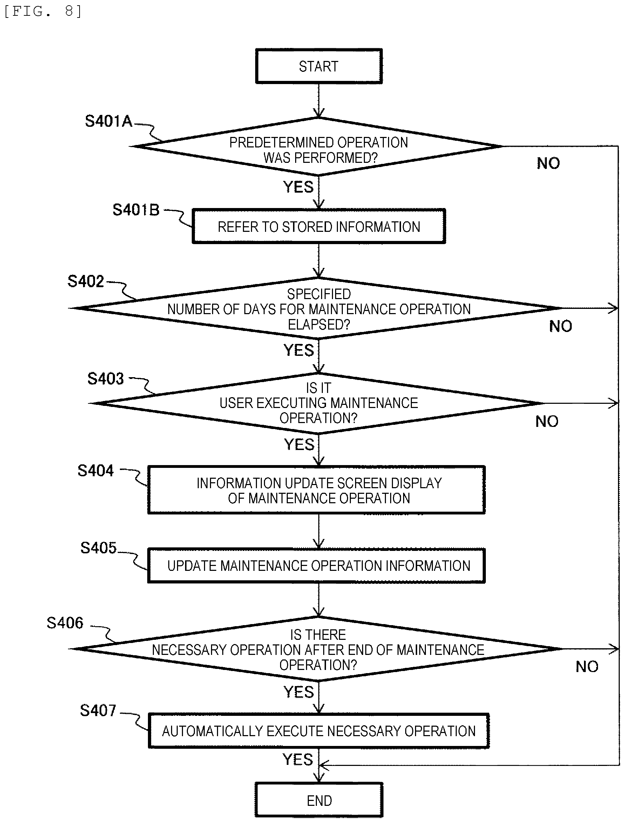

In the device state monitoring history storage unit 301b, the history of a device state is managed. For example, when the conditions that a cover is opened, an ISE cover is detached, and an ISE lever is opened are satisfied as a device state, the device state monitoring history storage unit 301b determines that ISE electrode replacement is performed. Accordingly, the information stored in the device state monitoring history storage unit 301b is updated, and is then reflected in a processing flow shown in FIG. 8.

### <Details of Maintenance Operation History Management Unit 302a and Maintenance Operation History Storage Unit 302b>

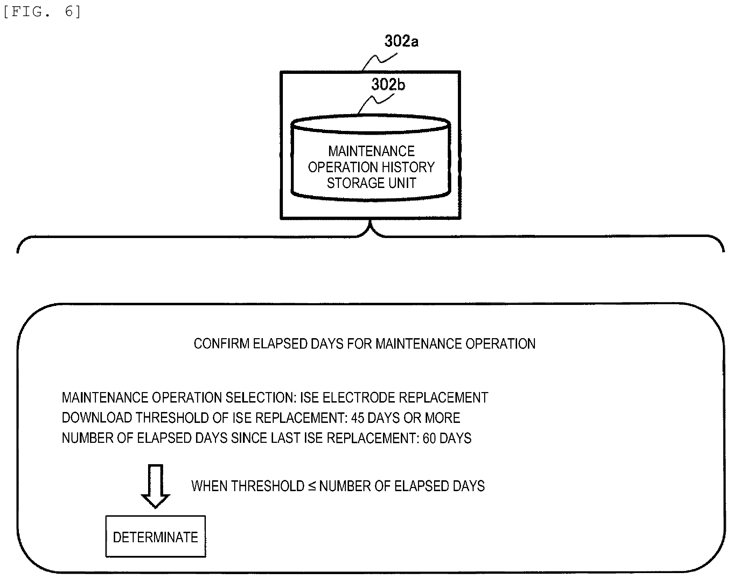

FIG. 6 is a diagram showing an example of a function of the maintenance operation history storage unit in FIG. 4. Information stored in the maintenance operation history storage unit 302b as shown in FIG. 6 is used to confirm the progress status of the specified number of days of the maintenance operation described with reference to FIG. 8.

When a maintenance operation is determined in the device state monitoring history storage unit 301b, an elapsed days threshold of the maintenance operation is downloaded inside the maintenance operation history storage unit 302b, and is compared with the number of elapsed days from the previous maintenance operation. When the threshold ≤ the number of elapsed days, the maintenance operation history storage unit 302b determines that the ISE electrode replacement is performed. Accordingly, the information stored in the maintenance operation history storage unit 302b is updated, and is then reflected in the processing flow shown in FIG. 8.

### <Details of User Information Management Unit 303a and User Information Storage Unit 303b>

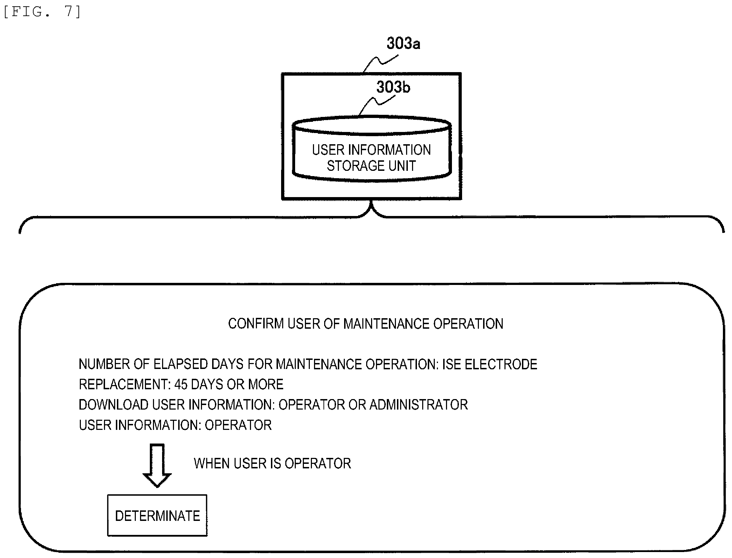

FIG. 7 is a diagram showing an example of a function of the user information storage unit in FIG. 4. Information stored in the user information storage unit 303b as shown in FIG. 7 is used to confirm the user of the maintenance operation described with reference to FIG. 8.

When a maintenance operation is determined in the maintenance operation history storage unit 302b, user information is confirmed inside the user information storage unit 303b. When the user is an operator or an administrator, the user information storage unit 303b determines that the ISE electrode replacement is performed. Accordingly, the information stored in the user information storage unit 303b is updated, and is then reflected in the processing flow shown in FIG. 8.

### <Details of Condition Determination Unit 304>

As shown in FIG. 4, the condition determination unit 304 includes a device state determination unit 304a, a history determination unit 304b, a user information determination unit 304c, an information update determination unit 304d, and an operation execution unit 304e, and is implemented by program processing using a processor, a memory, or the like.

FIG. 8 is a flowchart showing an example of processing contents in the condition determination unit 304 in FIG. 4.

In FIG. 8, first, the device state determination unit 304a of the condition determination unit 304 determines whether a predetermined operation was performed while the analysis unit 111 of FIG. 1 is stopped (step S401A). When it is determined that the predetermined operation was performed, the updated information stored in the device state monitoring history storage unit 301b in FIG. 4 is referred to accordingly (step S401B). More specifically, the predetermined operation includes various modes such as opening or closing information on the covers, detachment information on a cover of the ISE electrode, opening or closing information on the ISE electrode lever, and the present embodiment can be widely applied to these, and representative examples will be described later.

On the other hand, when it is determined in step S401A that the predetermined operation was not performed, the processing ends.

Subsequently, the history determination unit 304b of the condition determination unit 304 determines whether the information stored in the device state monitoring history storage unit 301b referred to in step S401B satisfies a maintenance operation execution days elapse condition stored in the maintenance operation history storage unit 302b (step S402). When it is determined in step S402 that the condition is not satisfied, the processing ends.

Subsequently, in FIG. 8, the user information determination unit 304c of the condition determination unit 304 determines whether the information stored in the maintenance operation history storage unit 302b referred to in step S402 satisfies a maintenance operation operator condition stored in the user information storage unit 303b (step S403). When it is determined in step S403 that the condition is not satisfied, the processing ends.

Next, in order to notify a user that it is determined that the maintenance operation is being performed, the information update determination unit 304d of the condition determination unit 304 notifies the user of an information update screen display of the maintenance operation confirming whether to update the maintenance operation information (step S404). Specifically, whether the maintenance operation was performed is confirmed, and a confirmation item for confirming whether to update the maintenance operation information is displayed on a screen or the like. An example of the screen is shown in FIGS. 9 and 10.

FIG. 9 shows a mode in which a confirmation area 510 for confirming whether to update the maintenance operation information is additionally displayed in a partial area of a maintenance screen 500 displayed on the display unit 131. By pressing the confirmation area 510, the maintenance operation information is updated.

FIG. 10 shows an example in which the maintenance operation information is displayed in a pop-up manner on the maintenance screen 500 displayed on the display unit 131, and the maintenance operation information is updated by checking a check box 610 of the maintenance operation update screen 600 to be displayed and pressing an OK button 611.

When the operation execution unit 304e of the condition determination unit 304 can confirm that the update of the maintenance operation information is selected by the user based on a confirmation screen as shown in FIGS. 9 and 10 (step S405), the operation execution unit 304e confirms whether there is a necessary operation after execution of the maintenance operation toward the recording unit 133 of the operation unit 130 (step S406). When it is determined that there is a necessary operation, the processing proceeds to step S407, and when it is determined that there is no operation, the processing ends.

Next, the operation execution unit 304e outputs an instruction signal to a corresponding device of the analysis unit 111 to execute a necessary operation after execution of the maintenance operation (step S407) .

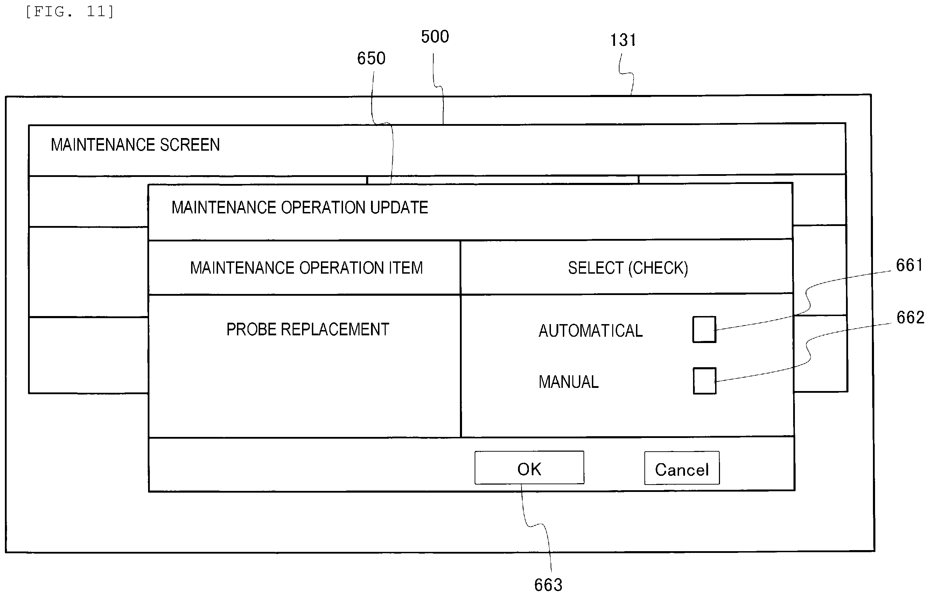

Here, regarding the necessary operation to be executed in step S407, after a result of the confirmation in step S405 is received, it is possible to request to select whether to automatically execute or manually execute the necessary operation after execution of the corresponding maintenance operation. As a method for requesting this selection, for example, a confirmation screen displayed on the display unit 131 as shown in FIG. 11 can be used. Selection is made by checking either a check box 661 for selecting automatic execution or a check box 662 for selecting manual execution on the displayed maintenance operation selection screen 650 and pressing an OK button 663.

Even when the necessary operation is set to be automatically executed, it may be difficult to automatically execute the necessary operation. Accordingly, it is possible to display a confirmation screen for confirming whether the necessary operation can be executed before being automatically executed.

As described above, in the condition determination unit 304 of the overall control unit 134, when it is detected that the operator executed a specific operation on the components (Yes in step S401A) and it is determined that a predetermined period elapsed after the previous specific operation was executed (Yes in step S402), control is performed to request confirmation as to whether execution status of the maintenance operation needs to be updated; or when it is detected that the operator executed a specific operation on the components (Yes in step S401A) and it is determined that a specific user is operating the automatic analyzer 100 (Yes in step S403), control is performed to request confirmation as to whether execution status of the maintenance operation needs to be updated.

All of the above-described steps S401A, S402, and S403 do not need to be provided, and any one or two steps can be provided. An order for the steps is not limited to the order shown in FIG. 8, and can be appropriately changed.

By using the method as described above, in a plurality of maintenance operation processes, it is possible to reduce the operation by forgetting to update the maintenance operation information after performing the maintenance operation of the automatic analyzer or automatically performing the necessary operation after the maintenance operation.

Next, a plurality of specific target portions will be listed below, but the invention is not limited thereto.

### <Probe Replacement>

When opening and closing of the covers of the automatic analyzer 100, attachment and detachment of the arm cover upper portion 402 of the reagent dispensing nozzle 116 shown in FIG. 2, and attachment and detachment of the reagent dispensing nozzle 116 are performed in the appropriate order while the analysis unit 111 in FIG. 1 is stopped, the device state determination unit 304a of the condition determination unit 304 refers to the updated information stored in the device state monitoring history storage unit 301b in FIG. 4 in response to an input from the opening and closing sensor 404 or the like (step S401A).

When 30 days or more elapsed after a previous probe replacement, the history determination unit 304b determines that the maintenance operation execution days elapse condition stored in the maintenance operation history storage unit 302b is satisfied (step S402).

When the operator is a general user or an administrator, the user information determination unit 304c determines that the maintenance operation operator condition stored in the user information storage unit 303b is satisfied (step S403).

The information update determination unit 304d displays a confirmation screen for confirming whether a probe replacement was performed on an interface in order to confirm with the user whether the probe replacement was performed as the maintenance operation (step S404).

When OK is selected (step S405), the operation execution unit 304e updates the maintenance operation information and executes a necessary operation (probe replacement) (step S406) after the maintenance operation (step S407).

### <ISE Electrode Replacement>

When opening and closing of the covers of the automatic analyzer 100, attachment and detachment of a cover of the ISE electrode, and opening and closing of an ISE electrode fixing lever 222 are performed in the appropriate order while the analysis unit 111 in FIG. 1 is stopped, the device state determination unit 304a of the condition determination unit 304 refers to the updated information stored in the device state monitoring history storage unit 301b in FIG. 4 in response to inputs from various sensors (step S401A).

When 45 days or more elapsed after a previous ISE electrode replacement, the history determination unit 304b determines that the maintenance operation execution days elapse condition stored in the maintenance operation history storage unit 302b is satisfied (step S402).

When the operator is a general user or an administrator, the user information determination unit 304c determines that the maintenance operation operator condition stored in the user information storage unit 303b is satisfied (step S403).

The information update determination unit 304d displays a confirmation screen for confirming whether an ISE electrode replacement was performed on the interface in order to confirm with the user whether the ISE electrode replacement was performed as the maintenance operation (step S404).

When OK is selected (step S405), the operation execution unit 304e displays an ISE electrode registration screen, instructs registration of the ISE electrode, updates the maintenance operation information, and executes a necessary operation (ISE check, reagent flow path prime) (step S406) after the maintenance operation (step S407).

### <Photometer Replacement>

When opening and closing of the covers of the automatic analyzer 100, opening and closing of a cover of the reaction disk 115, and attachment and detachment of a connector of a photometer of the colorimetric measurement unit 118 are performed in the appropriate order while the analysis unit 111 in FIG. 1 is stopped, the device state determination unit 304a of the condition determination unit 304 refers to the updated information stored in the device state monitoring history storage unit 301b in FIG. 4 in response to the input from the opening and closing sensor 404 or the like (step S401A).

When 30 days or more elapsed after a previous photometer replacement, the history determination unit 304b determines that the maintenance operation execution days elapse condition stored in the maintenance operation history storage unit 302b is satisfied (step S402).

When the operator is a general user or an administrator, the user information determination unit 304c determines that the maintenance operation operator condition stored in the user information storage unit 303b is satisfied (step S403).

The information update determination unit 304d displays a confirmation screen for confirming whether a photometer replacement was performed on the interface in order to confirm with the user whether the photometer replacement was performed as the maintenance operation (step S404).

When OK is selected (step S405), the operation execution unit 304e updates the maintenance operation information and executes a necessary operation (cell blank measurement) (step S406) after the maintenance operation (step S407).

Here, this operation can also be performed in an OFF state of device power. At this time, since the analysis unit 111 cannot be monitored when the power is OFF, the inputs from various sensors cannot be used as the determination condition.

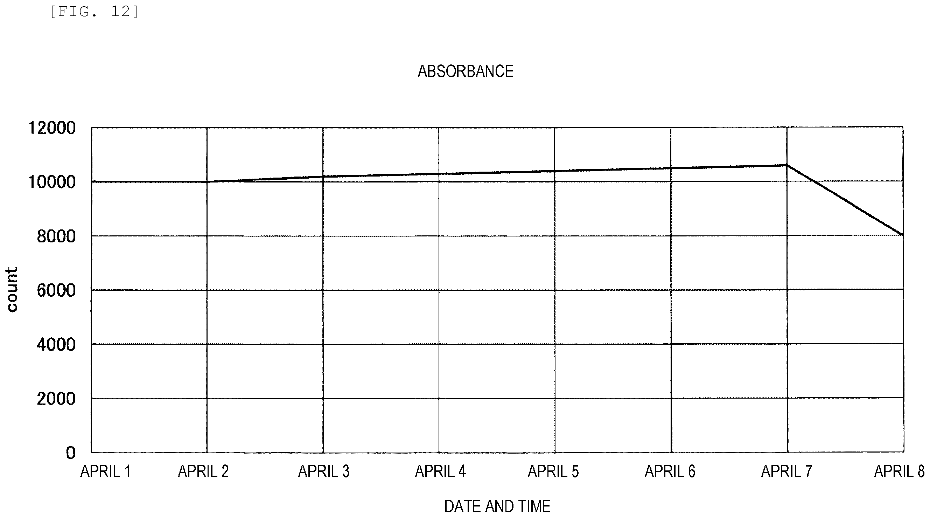

Therefore, in step S401A, instead of or in addition to the above-described processing, as shown in FIG. 12, when a change over time in a measurement result of the absorbance measured daily is confirmed and the measurement result decreases by 10% or more, processing for referring to the information stored in the device state monitoring history storage unit 301b in FIG. 3 can be performed.

### <Reaction Cell Replacement>

When opening and closing of the covers of the automatic analyzer 100 and attachment and detachment of the reaction cell 119 of the reaction disk 115 are performed in the appropriate order while the analysis unit 111 in FIG. 1 is stopped and the absorbance measurement result measured daily shown in FIG. 12 decreases by 10% or more, the device state determination unit 304a of the condition determination unit 304 refers to the updated information stored in the device state monitoring history storage unit 301b in FIG. 4 in response to the input from the opening and closing sensor 404 or the like (step S401A).

When 20 days or more elapsed after a previous replacement of the reaction cell 119, the history determination unit 304b determines that the maintenance operation execution days elapse condition stored in the maintenance operation history storage unit 302b is satisfied (step S402) .

When the operator is a general user or an administrator, the user information determination unit 304c determines that the maintenance operation operator condition stored in the user information storage unit 303b is satisfied (step S403).

The information update determination unit 304d displays a confirmation screen for confirming whether a reaction cell replacement was performed on the interface in order to confirm with the user whether the replacement of the reaction cell 119 was performed as the maintenance operation (step S404).

When OK is selected (step S405), the operation execution unit 304e updates the maintenance operation information and executes a necessary operation (cell blank measurement) (step S406) after the maintenance operation (step S407).

Next, effects of the present embodiment will be described.

The automatic analyzer 100 that analyzes a specimen according to the present embodiment described above includes: the analysis unit 111 including a plurality of components and configured to analyze the specimen, and the overall control unit 134 configured to control an operation of the analysis unit 111. The analysis unit 111 is provided with sensors that monitor states of the components, and when the sensors detect that an operator executed a specific operation on the components, the overall control unit 134 requests confirmation as to whether execution status of a maintenance operation needs to be updated.

In a device configuration in the related art, there was a problem that, for example, when a user forgets to perform update processing although performed a maintenance operation, the device issues an alarm or the like based on old information, and thus may perform maintenance again uselessly.

However, according to the invention, since it is possible to determine whether the maintenance operation is automatically executed on a device side and to confirm with the user whether the maintenance operation is reflected in the history, even when the user executes the maintenance operation at a convenient timing, it is possible to automatically recognize the maintenance operation and to confirm with the user whether to reflect the maintenance operation in the history. Therefore, it is easy to reflect the maintenance operation, and the latest and accurate information can be surely registered in the device, and thus it is possible to greatly reduce the omission of reflection.

When it is detected that the operator executed a specific operation on the components and it is determined that a predetermined period elapsed after the previous specific operation was executed, or when it is detected that the operator executed a specific operation on the components and it is determined that a specific user is operating the automatic analyzer 100, the overall control unit 134 can confirm with the user whether to surely reflect the operation status of the maintenance operation in the history by requesting confirmation as to whether the execution status of the maintenance operation needs to be updated.

Furthermore, by detecting at least one of whether various covers of the automatic analyzer 100 are opened or closed and attached or detached, whether the operator accesses the analysis unit 111 from the end of the analysis operation to the start of the analysis preparation operation, and whether the components are attached or detached, the sensors can more reliably detect whether the maintenance operation of a target was accurately performed, and can reflect the latest and more accurate information in the device.

After a result of the confirmation is received, by requesting the user to select whether to automatically execute or manually execute the necessary operation after the execution of the corresponding maintenance operation, it is possible to determine whether to perform post-processing of the maintenance operation by a more convenient method for the user.

Furthermore, by further providing the display unit 131 that requests confirmation of the update, it is possible to confirm whether to reflect the maintenance operation to the user in a more understandable manner.

### <Others>

The invention is not limited to the embodiment described above, and various modifications and applications are possible. The above-described embodiment is described in detail in order to make the invention easy to understand, and the invention is not necessarily limited to those have all the configurations described.

### Reference Signs List

- 100: automatic analyzer
- 101: conveyance unit
- 102: rack supply tray
- 103: rack buffer
- 104: specimen rack
- 106: conveyance line
- 107: rack storage tray
- 108: specimen information reading device
- 111: analysis unit
- 112: specimen dispensing line
- 114: electrolyte measurement unit
- 115: reaction disk
- 116: reagent dispensing nozzle
- 117: reagent disk
- 118: colorimetric measurement unit
- 119: reaction cell
- 120: reagent bottle
- 130: operation unit
- 131: display unit (display device)
- 132: input unit
- 133: recording unit
- 134: overall control unit (control unit)
- 134a: conveyance unit controller
- 134b: analysis unit controller
- 134c: operation recording unit
- 201: dilution tank
- 202: specimen dispensing nozzle
- 203: diluted solution dispensing nozzle
- 204: internal standard solution dispensing nozzle
- 205: specimen solution aspiration nozzle
- 206: pipe
- 207: sodium ion selective electrode
- 208: potassium ion selective electrode
- 209: chlorine ion selective electrode
- 210: reference electrode
- 211: pipe
- 212: sipper pump
- 213: potential measurement unit
- 214: diluted solution container
- 215: internal standard solution container
- 216: temperature control unit
- 217: comparison electrode solution container
- 218: diluted solution pump
- 219: internal standard solution pump
- 220: valve
- 221: waste solution portion
- 222: ISE electrode fixing lever
- 301a: device state history management unit
- 301b: device state monitoring history storage unit
- 302a: maintenance operation history management unit
- 302b: maintenance operation history storage unit
- 303a: user information management unit
- 303b: user information storage unit
- 304: condition determination unit
- 304a: device state determination unit
- 304b: history determination unit
- 304c: user information determination unit
- 304d: information update determination unit
- 304e: operation execution unit
- 401: vertical rotation mechanism
- 402: arm cover upper portion
- 403: arm cover lower portion
- 404: opening and closing sensor
- 500: maintenance screen
- 510: confirmation area
- 600: maintenance operation update screen
- 610, 661, 662: check box
- 611, 663: OK button
- 650: maintenance operation selection screen

## Claims

1. An automatic analyzer for analyzing a specimen, the automatic analyzer comprising:
an analysis unit including a plurality of components and configured to analyze the specimen; and
a control unit configured to control an operation of the analysis unit, wherein
the analysis unit is provided with a sensor that monitors states of the components, and
when the sensor detects that an operator executed a specific operation on the components, the control unit requests confirmation as to whether execution status of a maintenance operation needs to be updated.

2. The automatic analyzer according to claim 1, wherein
when it is detected that the operator executed the specific operation on the components and it is determined that a predetermined period elapsed after the specific operation was executed last time, the control unit requests confirmation as to whether the execution status of the maintenance operation needs to be updated.

3. The automatic analyzer according to claim 1, wherein
when it is detected that the operator executed the specific operation on the components and it is determined that a specific user is operating the automatic analyzer, the control unit requests confirmation as to whether the execution status of the maintenance operation needs to be updated.

4. The automatic analyzer according to claim 1, wherein
the sensor detects at least one of whether various covers of the automatic analyzer are opened or closed and attached or detached, whether the operator accesses the analysis unit from an end of an analysis operation to a start of an analysis preparation operation, and whether the components are attached or detached.

5. The automatic analyzer according to claim 1, wherein the control unit requests to select whether to automatically execute or manually execute a necessary operation after the execution of the maintenance operation after receiving a result of the confirmation.

6. The automatic analyzer according to claim 1, further comprising:
a display device configured to request confirmation of the update.

7. A maintenance method for an automatic analyzer for analyzing a specimen, the maintenance method comprising:
a monitoring step of monitoring, by a sensor provided in an analysis unit that analyzes the specimen, a state of a component constituting the analysis unit;
a confirmation step of, when it is detected in the monitoring step that an operator executed a specific operation on the component, requesting confirmation as to whether execution status of a maintenance operation needs to be updated.
